# EUROPEAN PATENT SPECIFICATION

(11) **EP 4 225 404 B1**
(45) Date of publication and mention of the grant of the patent: **18.02.2026**
(21) Application number: 21787240.7
(22) Date of filing: 15.09.2021
(51) Int. Cl.: A61M 5/20, A61M 5/315

(54) **AUTO-INJECTOR SYSTEM**
AUTO-INJEKTOR-SYSTEM
SYSTEME D'AUTO-INJECTEUR

(30) Priority: 07.10.2020 US 202063088515 P
(43) Date of publication of application: 16.08.2023
(73) Proprietor: Bayer Healthcare LLC, Whippany, NJ 07981 (US)
(72) Inventor: STOUT, Christopher, Pleasanton, CA 94566 (US); CRUZADA, Julian, Oakland, CA 94602 (US)
(74) Representative: BIP Patents
(86) International application number: PCT/US2021/050355
(87) International publication number: WO 2022/076138

(56) References cited:
- US-A1- 2008 039 789
- US-A1- 2013 317 428
- US-A1- 2016 175 526

## Description

### Background

The pharmaceutical industry uses various auto-injectors with pre-filled syringes (PFS). Auto-injector (Al) manufacturers develop AIs for a specific solution volume. If the solution volume requirement changes during development of the AI, then a portion of the AI development process needs to be repeated or changed. This makes the use of auto-injectors in early clinical trials problematic due to the length of time needed to develop the AI with a new solution volume. Further, the dose patients need may be based on their weight. Therefore, there is a need for an AI system and method which uses a pre-filled syringe filled at maximum capacity but has the ability to inject various different volumes of solution.

Further, there is a need for an auto-injector system and method which employs pre-filled syringes with various fill volumes where the AI can inject using all of the various filled pre-filled syringes.

Still further, there is a need for an AI system and method which can easily integrate with various pre-filled syringes and syringe volumes using the same or similar parts. There have been attempts in the prior art to address at least some of these problems: US2008/0039789 A1 discloses a reloadable medicine injector in which a barrel with a receiving cavity is adapted to slidably receive a syringe subassembly for axial movement therein. Upon removal of a safety and release of a syringe driver, the syringe driver moves forward and injects the syringe needle. The reloadable medicine injector is capable of use for single or for multiple injections. To enable such use, one or more stops in the form of dose stop collars can be releasably mounted to the driver or to the plunger rod. The collar and possible multiple such collars are advantageously positioned in the forward path of the headed end of the plunger rod. Collar or collars stop forward motion of the plunger rod at such point where a selected first dosage has been expelled from the syringe subassembly.

US20160175526 A1 discloses a drive unit for a drug delivery device comprising a carrier, a plunger arranged within the carrier and slidable in a direction of a longitudinal axis (A), a drive spring arranged for biasing the plunger in a distal direction (D) relative the carrier, and a drive force adaption mechanism for modifying a force exerted by the drive spring on the plunger.

US20130317428 A1 discloses an auto-injector for administering a dose of a liquid medicament (M) having a tubular chassis telescopable in an elongate chassis, a carrier subassembly comprising a tubular carrier slidably arranged partially inside the chassis and inside the case, where the carrier is adapted to contain a syringe with a hollow injection needle, a drive spring and a plunger for forwarding load of the drive spring to a stopper of the syringe, wherein the syringe is lockable for joint axial translation with the carrier. Changing the length of the plunger is disclosed for injecting different volumes.

The cited and other problems experienced in the art have been addressed and obviated by the present invention.

### Summary

To accomplish these objectives, the present invention provides an auto-injector system for injecting a predetermined solution volume in one shot from a pre-filled syringe according to claim 1. Further embodiments are defined in the dependent claims.

### Brief Description of the Drawings

The skilled artisan will understand that the drawings, described below, are for illustration purposes only. The drawings are not intended to limit the scope of the present teachings or claims in any way.
FIG. 1 - This figure shows a general perspective of an auto-injector system.
FIG. 2 - This figure shows an exploded view of a first embodiment of an auto-injector system with pre-filled syringe.
FIG. 3 - This figure shows a cross-sectional view of the first embodiment of the auto-injector system with pre-filled syringe.
FIG. 4 -This figure shows an exploded view of a second embodiment of an auto-injector system with pre-filled syringe.
FIG. 5 -This figure shows a cross-sectional view of a second embodiment of the auto-injector system with pre-filled syringe.
FIG. 6 - This figure shows an exploded view of a third embodiment of an auto-injector system with pre-filled syringe.
FIG. 7 - This figure shows a cross-sectional view of the third embodiment of the auto-injector system with pre-filled syringe.
FIG. 8 - This figure shows comparisons of various pre-filled syringes for an auto-injector system with (A) varying interchangeable plunger rod extensions; and (B) varying interchangeable plunger rods.

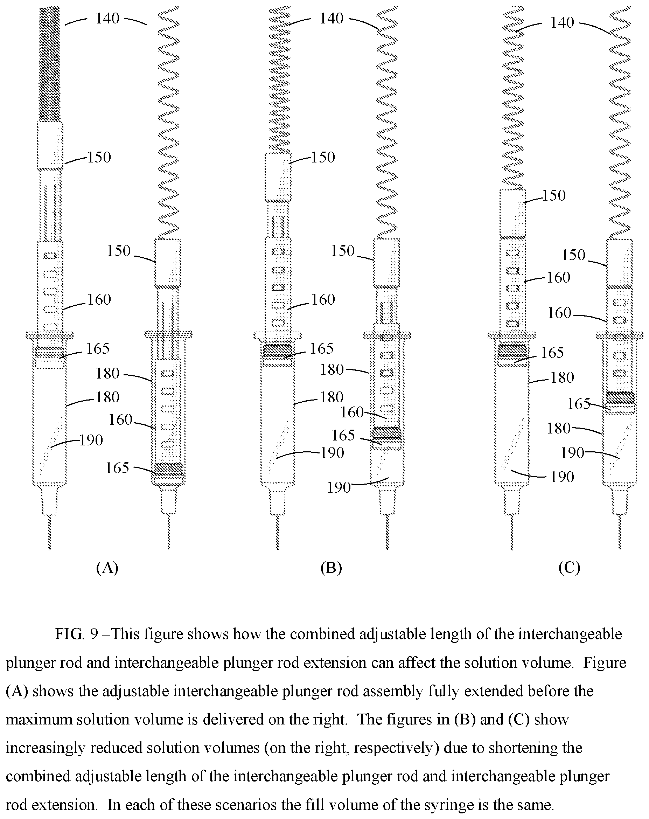

FIG. 9 - This figure shows how the combined adjustable length of the interchangeable plunger rod and interchangeable plunger rod extension can affect the solution volume. Figure (A) shows the adjustable interchangeable plunger rod assembly fully extended before the maximum solution volume is delivered on the right. The figures in (B) and (C) show increasingly reduced solution volumes (on the right, respectively) due to shortening the combined adjustable length of the interchangeable plunger rod and interchangeable plunger rod extension. In each of these scenarios the fill volume of the syringe is the same.
FIG. 10A - This figure shows the first step of the not-claimed method of injection using the present embodiments.
FIG. 10B - This figure shows the second step of the not-claimed method of injection using the present embodiments.

### Detailed Description

This disclosure describes embodiments related to pre-filled syringes and methods (not claimed) of using them.

For the purpose of interpreting this specification, the following definitions will apply. In the event that any definition set forth below conflicts with the usage of that word in any other document, the definition set forth below shall always control for purposes of interpreting this specification and its associated claims unless a contrary meaning is clearly intended (for example in the document where the term is originally used).

Whenever appropriate, terms used in the singular will also include the plural and vice versa. The use of "a" herein means "one or more" unless stated otherwise or where the use of "one or more" is clearly inappropriate.

The use of "or" means "and/or" unless stated otherwise. The use of "comprise," "comprises," "comprising," "include," "includes," and "including" are interchangeable and are not limiting. The terms "such as," "for example," and "e.g." also are not intended to be limiting. For example, the term "including" shall mean "including, but not limited to."

As used herein, the term "about" refers to +/- 10% of the unit value provided.

As used herein, the term "substantially" refers to the qualitative condition of exhibiting a total or approximate degree of a characteristic or property of interest. One of ordinary skill in the biological arts will understand that biological and chemical phenomena rarely, if ever, achieve or avoid an absolute result because of the many variables that affect testing, production, and storage of biological and chemical compositions and materials, and because of the inherent error in the instruments and equipment used in the testing, production, and storage of biological and chemical compositions and materials. The term "substantially" is, therefore, used herein to capture the potential lack of completeness inherent in many biological and chemical phenomena.

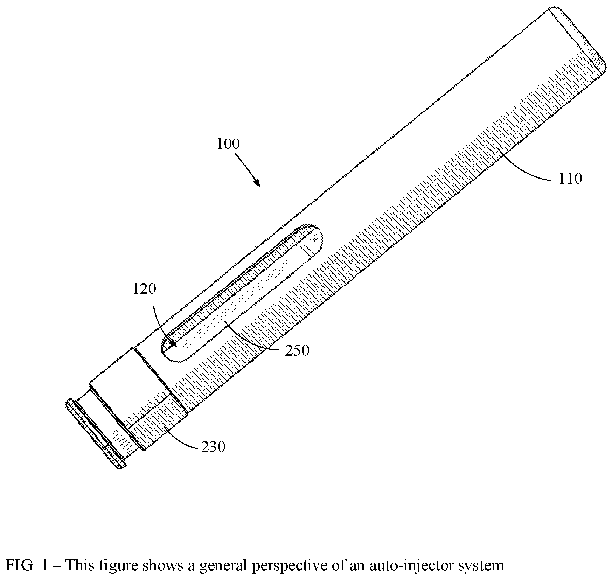

Referring now to the figures, FIG. 1 shows a general perspective of an auto-injector system 100. The auto-injector system 100 external parts comprise an auto-injector housing 110 and an optional auto-injector cap 230. The auto-injector housing 110 comprises an auto-injector housing window 120 for viewing a solution volume 250. The auto-injector housing 110 can comprise any number of materials including but not limited to plastic, metal, composite or other similar type materials known or used in the art. The housing window 120 can be designed in any number of shapes or sizes to allow a user to view the solution volume 250 in the auto-injector system 100. The optional auto-injector cap 230 can also be designed in any number of shapes, sizes, or designs known in the art. The auto-injector system 100 can be designed for one or more injection runs.

The optional auto-injector cap 230 contacts or can be removably attached to the auto-injector housing 110. In most embodiments the optional auto-injector cap 230 will be designed for ease of removal. Various options for joining the optional auto-injector cap 230 to the syringe cap 220 and/or auto-injector housing 110 include and are not limited to the use of a set of threads for mating the ends, attachment via clips or fasteners or any other types of devices or means known in the art. The optional auto-injector cap 230 can be designed of plastic, metal, composite or similar material know or used in the art. The optional auto-injector cap 230 is positioned and disposed at the injection end of the auto-injector system 100.

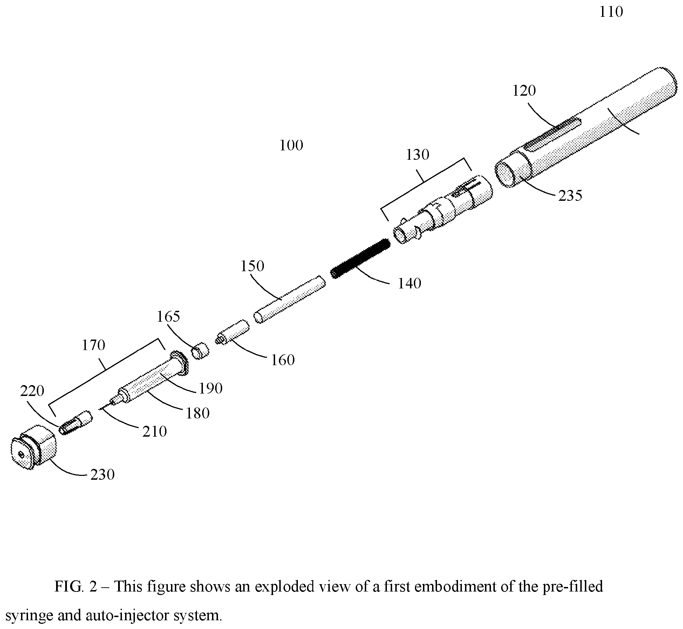

FIG. 2 shows an exploded view of a first embodiment of the auto-injector system 100 including the external parts, internal parts, and how they assemble together. The auto-injector system 100 comprises, an auto-injector housing 110, a release mechanism 130, a compression spring 140, an interchangeable plunger rod 150, an interchangeable plunger rod extension 160, a plunger cap 165, a pre-filled syringe 170, a syringe cap 220, and an optional auto-injector cap 230. The auto-injector housing 110 and the optional auto-injector cap 230 enclose the release mechanism 130, the spring 140, the interchangeable plunger rod 150, the interchangeable plunger rod extension 160, the plunger cap 165, the pre-filled syringe 170, and the syringe cap 220.

The auto-injector system 100 is designed for housing the release mechanism 130 and spring 140 which is used for compressing and injecting a solution volume 250 which is placed in the auto-injector system 100 (typically inside the pre-filled syringe 170 and the syringe chamber 190). The release mechanism 130 is not shown in detail in the drawings for ease of viewing since there are many possible designs and orientations for the actuating parts and components. The release mechanism 130 is simply used for actuating the device and injection when the device is actuated by a user to make an injection. Various springs, components, and mating parts may be employed cooperatively and efficiently to accomplish this purpose. Various different parts and release mechanisms 130 are known and used in the art.

The spring 140 can comprise plastic, metal, an alloy, or other similar type material known or used in the art. Various shapes, sizes, and quantities may be employed with the present embodiments. The spring 140 is particularly designed to keep the internal parts under compression so that when the release mechanism 130 is actuated the motion or force is translated to the other components to actuate the injection and inject the solution volume. In other words, the parts define a continuous auto-injector system for injecting various solution volumes.

The interchangeable plunger rod 150 can comprise various shapes, designs, and materials known or used in the art including but not limited to plastic, resin, metal, and composite materials. The interchangeable plunger rod 150 can be hollow or solid. The interchangeable plunger rod 150 lengths can vary or be fixed depending upon the pre-filled syringe and embodiment. Preferable length of the interchangeable plunger rod 150 is from about 5 to 80 millimeters. Other sizes, shapes, and lengths are within the anticipated scope of the present embodiments. The interchangeable plunger rod 150 can be retained or replaced in certain other injections for different patients. For instance, in certain embodiments the interchangeable plunger rod 150 can be replaced with a second interchangeable plunger rod 150' having a second interchangeable plunger rod length. In other embodiments, the interchangeable plunger rod 150 can be maintained using the same interchangeable plunger rod 150 and providing the defined interchangeable plunger rod length.

The interchangeable plunger rod extension 160 can comprise various shapes, designs, and materials know or used in the art including, but not limited to plastic, resin, metal, and composite materials. The interchangeable plunger rod extension 160 can be solid or hollow. The interchangeable plunger rod extension 160 lengths can vary or be fixed depending upon the pre-filled syringe and embodiment. Preferable lengths of the interchangeable plunger rod extension 160 are from about 5 to 80 millimeters. Other sizes, shapes, and lengths are within the anticipated scope of the present embodiments. The interchangeable plunger rod extension 160 can be replaced or changed in a different injection run depending upon the embodiment. For instance, in a different injection run the interchangeable plunger rod extension 160 can be replaced with a second interchangeable plunger rod extension 160' having a second interchangeable plunger rod extension length. In other embodiments, the interchangeable plunger rod extension 160 can be maintained with the defined interchangeable plunger rod extension length.

The plunger cap 165 is typically associated with the pre-filled syringe. It is described separately here since it represents an important component related to the embodiments of the invention. It can comprise a portion of the interchangeable plunger rod extension 160, interchangeable plunger rod 150, or serve as a separate stand-alone component in contact with one or more of these components. The plunger cap 165 can comprise rubber or any similar type of material known or used in the art. A portion or side of the plunger cap 165 is typically designed to contact a solution for injection such as solution volume 250. A force can be supplied to the plunger cap 165 via interchangeable plunger rod 150, interchangeable plunger rod extension 160 or any other parts known or provided in an embodiment. The force is used to slide plunger cap 165 to compress and inject or inject the solution volume 250 from the pre-filled syringe 170. The plunger cap 165 can be varied or a fixed length and width. Preferably the length of plunger cap 165 is fixed and can be in a length of about 5 to 6 millimeters. Other sizes and shapes known and used in the art can be employed with the present embodiments. The plunger cap 165 is positioned within the syringe chamber 190 by the filling company based on the solution volume 250. The plunger cap 165 is placed adjacent to the solution volume 250 within the syringe chamber 190. The plunger cap 165 can be used or employed in a number of injection runs. In additional injection runs the plunger cap 165 is typically retained and not replaced or changed. However, it could be imagined in certain embodiments that a new or different plunger cap 165 or 165' could be employed.

The pre-filled syringe 170 comprises a syringe barrel 180, a syringe chamber 190, a solution volume 250, a plunger cap 165, a syringe needle 210, and a syringe cap 220. The pre-filled syringe 170 is designed to fit within the auto-injector housing 110. Various pre-filled syringes 170 known in the art can be used with the present auto-injector system 100. The types and sizes of the pre-filled syringes 170 include and are not limited to the following: 0.5 mL, 1.0 mL, 1.5 mL, and 2.0 mL. The pre-filled syringe 170 can comprise plastic or other similar type material used or known in the art. Other shapes, sizes, and designs are possible which are known and used in the art and which can be employed with the present embodiments. The use of a pre-filled syringe 170 provides for an unexpected benefit of not requiring complete part replacement or delay for set up of auto-injector system 100. In certain other embodiments it could be further envisioned that the pre-filled syringe 170 could also be replaced or changed if necessary or desirable.

The syringe barrel 180 and the syringe chamber 190 can vary in shape, size, and volume. Preferable volumes include and are not limited to 0.5 mL, 1.0 mL, 2.0 mL. Other volumes not described which are larger, smaller, or within the above described range are within the scope of the present embodiments. The syringe barrel 180 can comprise plastic, metal, composite material or any other known or unknow material which is used in the art.

The syringe needle 210 is used for injecting the solution volume 250. The syringe needle 210 serves both to puncture a user's skin and to deliver the solution volume 250 through subcutaneous delivery. It can be anticipated that the syringe needle 210 could also be employed to deliver the solution volume 250 in other ways or designs known or not known in the art. The syringe needle 210 can comprise metal, composite, alloy or other similar types of materials known or used in the art.

The syringe cap 220 is used to cover the end of the syringe needle 210. The syringe cap 220 along with the auto-injector cap 230 prevent needle sticks when the auto-injector system 100 is not in use. The syringe cap 220 can comprise plastic, metal, or other composite material known or used in the art. Various shapes and designs of the syringe cap 220 can also be anticipated with the present embodiments.

The syringe chamber 190 is a fixed volume. However, the solution volume 250 can vary in amount and composition within the syringe chamber 190. The position of the plunger cap 165 is set in a pre-filled syringe determined by the solution volume 250. The position of the plunger cap 165 is typically set adjacent to the solution volume 250 by the user or filling manufacturer. An auto-injector is designed to inject a set solution volume 250 based on the position of the plunger cap 165 within the syringe chamber 190. If the solution volume 250 is changed by the filling manufacturer within the pre-filled syringe chamber 190 and, therefore, the plunger cap 165 position is changed, the appropriate size interchangeable plunger rod extension 160 can be used in combination with the interchangeable plunger rod 150 to fill the void left by the changed plunger cap 165 position. This provides for increased flexibility in auto-injector systems that was not possible in the past.

The solution volume 250 can comprise any solution, drug, or biologic which would be known or delivered in the pre-filled syringe or biological arts for subcutaneous injection. For instance, various drugs, biologics, and solutions can be delivered using the embodiments of the invention. Other anticipated drugs or therapeutics used for delivery could include those molecules used or developed in the fields of Oncology, gene therapy and cell therapy. Solution volume 250 can also be changed or adjusted during additional or multiple injection runs. For instance, it can be imagined in a different injection run if one or more of the components are changed or adjusted, the solution volume 250 could be changed or adjusted to inject a second solution volume 250'.

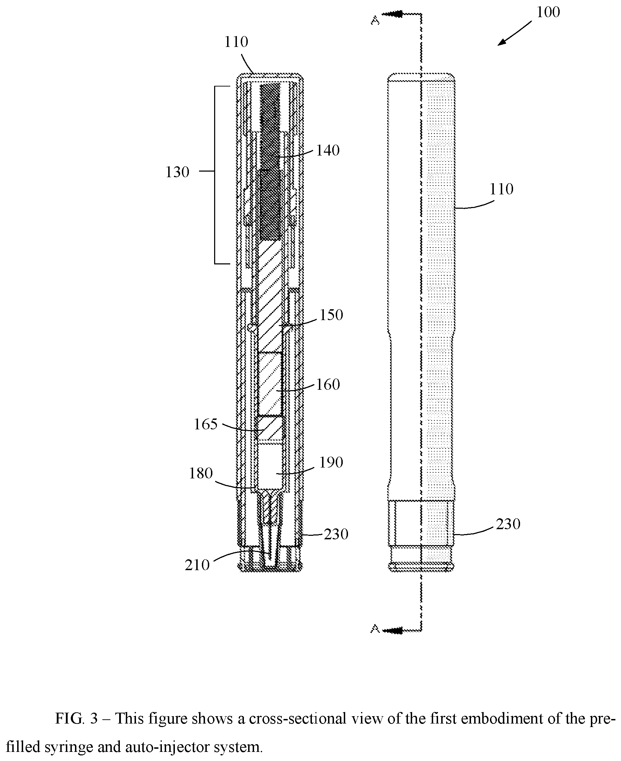

FIG. 3 shows a cross-sectional view of the first embodiment of the auto-injector system 100 with pre-filled syringe 170. Of particular note in this embodiment is the positioning of the interchangeable plunger rod 150, the interchangeable plunger rod extension 160, and the plunger cap 165. In this embodiment the interchangeable plunger rod extension 160 that's interchangeable is designed to be positioned adjacent to the interchangeable plunger rod 150 that's interchangeable. In the first embodiment of the invention, the interchangeable plunger rod extension 160 is interposed between the interchangeable plunger rod 150 and the plunger cap 165. Typically, the plunger cap 165 has a constant length as shown in Table 1. However, it can be imagined in certain embodiments that its length could be varied.

Table 1 shows the various configurations of components of the first embodiment of the invention using a 1.0 mL capacity pre-filled syringe 170. In the first embodiment the interchangeable plunger rod 150 length is maintained at a constant 50 mm, the interchangeable plunger rod extension 160 is changed in length, and the syringe chamber 190 is kept constant at 1.0 mL but is filled with various fill volumes as shown in the table. This arrangement and orientation allow a manufacturer to quickly and effectively adjust certain components to inject various solution volumes 250 or 250'.

| Table - 1 | 1.0mL Capacity Syringe | | | |
|---|---|---|---|---|
| Plunger Cap Length (mm) | 5 | 5 | 5 | 5 |
| Interchangeable Plunger Rod Extension Length (mm) | 0 | 8 | 16 | 24 |
| Interchangeable Plunger Rod Length (mm) | 50 | 50 | 50 | 50 |
| Combined Length (Plunger Cap + Interchangeable Plunger Rod+ Interchangeable Plunger Rod Extension) (mm) | 55 | 63 | 71 | 79 |
| Chamber Capacity Volume (mL) | 1.0 | 1.0 | 1.0 | 1.0 |
| Fill/Solution Volume (mL) | 1.0 | 0.75 | 0.5 | 0.25 |

As shown in Table 1 it can be observed in a 1.0 mL capacity syringe that various solution volumes (based on fill volume) of 1.0, 0.75, 0.5, and 0.25 mL can be injected from an AI comprising that same 1.0 mL capacity pre-filled syringe 170 while replacing the interchangeable plunger rod extension 160 with lengths of 0, 8, 16, and 24 millimeters, while the interchangeable plunger rod 150 length is fixed at 50 millimeters. The ability to inject various amounts of solution volume 250 or 250'makes it a very flexible and efficient system without the need for redesigning and redeveloping another version of the AI when a different solution volume 250 or 250' is required.

Table 2 shows similar data and results but using a larger 2.0 mL pre-filled syringe 170 and method.

| Table -2 | 2.0mL Capacity Syringe | | | |
|---|---|---|---|---|
| Plunger Cap Length (mm) | 5 | 5 | 5 | 5 |
| Interchangeable Plunger Rod Extension Length (mm) | 0 | 10 | 20 | 30 |
| Interchangeable Plunger Rod Length (mm) | 50 | 50 | 50 | 50 |
| Combined Length (Plunger Cap + Interchangeable Plunger Rod + Interchangeable Plunger Rod Extension) (mm) | 55 | 65 | 75 | 85 |
| Chamber Capacity Volume (mL) | 2.0 | 2.0 | 2.0 | 2.0 |
| Fill/Solution Volume (mL) | 2.0 | 1.5 | 1.0 | .5 |

Table 2 shows a 2.0 mL capacity pre-filled syringe 170 with various solution volumes 250 (Fill/Solution Volume) of 2.0, 1.5, 1.0, and 0.5 mL Again the interchangeable plunger rod 150 length and syringe chamber 190 volumes are fixed at 50 mL and 2.0 mL respectively. The interchangeable plunger rod extension 160 lengths are 0, 10, 20, and 30 mm respectively.

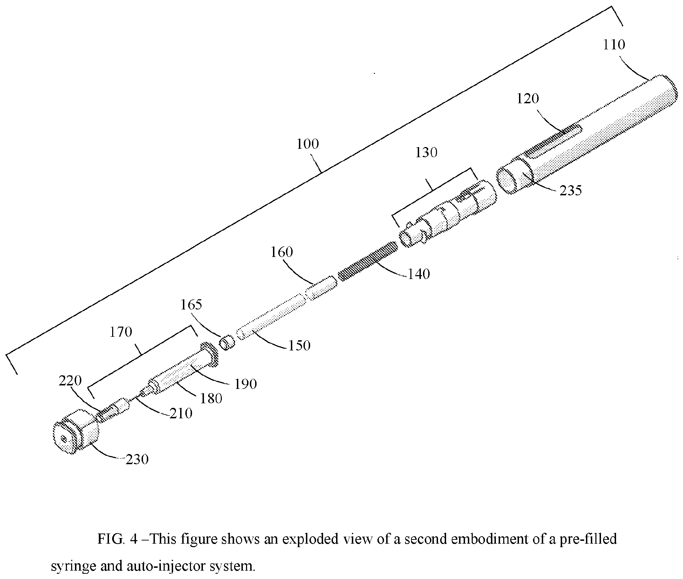

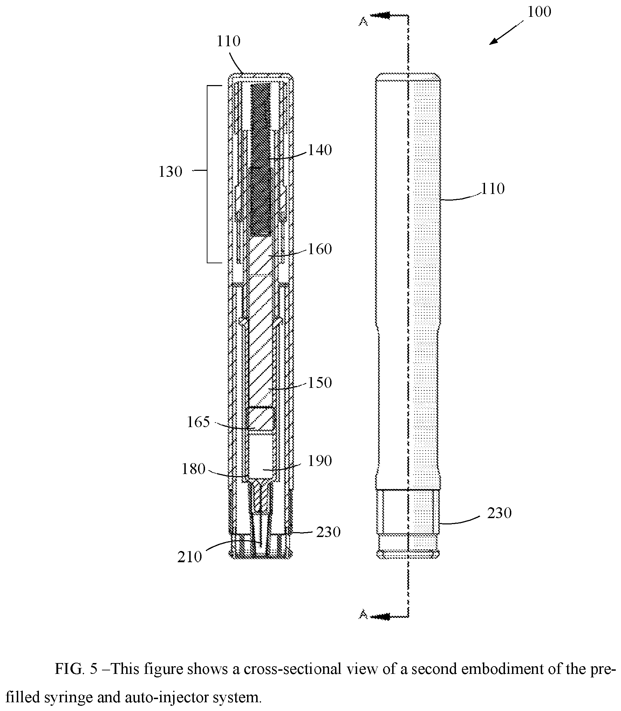

FIG. 4 and FIG 5 show a second embodiment of the invention. In this embodiment of the invention the interchangeable plunger rod extension 160 is interposed between the interchangeable plunger rod 150 and the spring 140.

| Table - 3 | 1.0mL Capacity Syringe | | | |
|---|---|---|---|---|
| Plunger Cap Length (mm) | 5 | 5 | 5 | 5 |
| Interchangeable Plunger Rod Extension Length (mm) | 10 | 10 | 10 | 10 |
| Interchangeable Plunger Rod Length (mm) | 40 | 48 | 56 | 64 |
| Combined Length (Plunger Cap + Interchangeable Plunger Rod+ Interchangeable Plunger Rod Extension) (mm) | 55 | 63 | 71 | 79 |
| Chamber Capacity Volume (mL) | 1.0 | 1.0 | 1.0 | 1.0 |
| Fill/Solution Volume (mL) | 1.0 | 0.75 | 0.5 | 0.25 |

As shown in Table 3 it can be observed in a 1.0 mL capacity syringe that various solution volumes of 1.0, 0.75, 0.5, and 0.25 mL can be injected from the a 1.0 mL capacity syringe while using varying interchangeable plunger rod 150 lengths of 40, 48, 56, 64 mm, while the interchangeable plunger rod extension 160 length is fixed at 10 mm. The ability to inject various amounts of solution volume 250 makes it a very flexible and efficient system without the need for frequent system changes or adjustments when a different solution volume 250 or 250' is desired.

| Table -4 | 2.0mL Capacity Syringe | | | |
|---|---|---|---|---|
| Plunger Cap Length (mm) | 5 | 5 | 5 | 5 |
| Interchangeable Plunger Rod Extension Length (mm) | 10 | 10 | 10 | 10 |
| Interchangeable Plunger Rod Length (mm) | 40 | 50 | 60 | 70 |
| Combined Length (Plunger Cap + Interchangeable Plunger Rod + Interchangeable Plunger Rod Extension) (mm) | 55 | 65 | 75 | 85 |
| Chamber Capacity Volume (mL) | 2.0 | 2.0 | 2.0 | 2.0 |
| Fill/Solution Volume (mL) | 2.0 | 1.5 | 1.0 | .5 |

Table 4 shows a 2.25 mL capacity syringe with various solution volumes of 2.0, 1.5, 1.0, and 0.5 mL. The interchangeable plunger rod extension 160 length and syringe chamber 190 volumes are fixed at 10 mm and 2.0 mL respectively. The interchangeable plunger rod 150 lengths are 40, 50, 60 and 70 mm respectively.

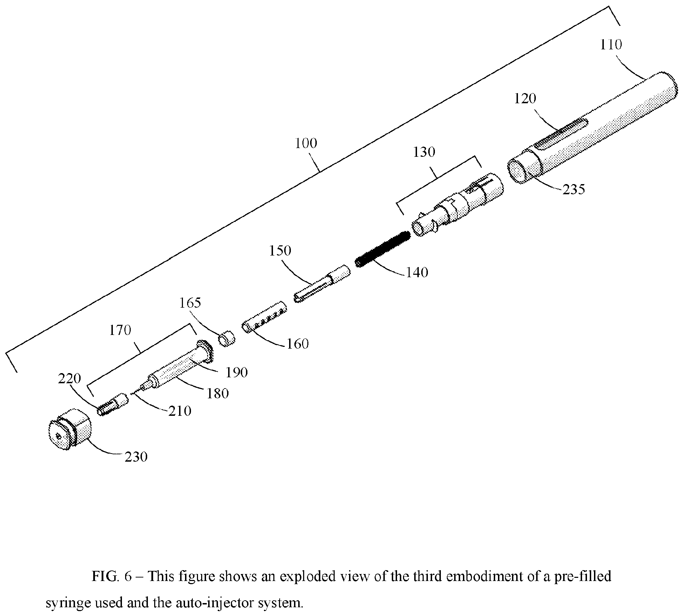

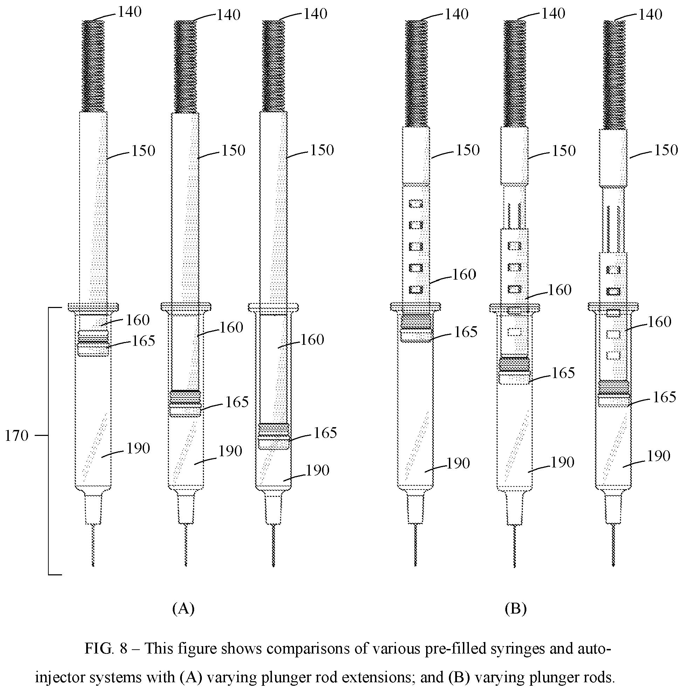

FIG. 6 shows an exploded view of a third embodiment of the invention. In this embodiment the combined length of the interchangeable plunger rod 150 and interchangeable plunger rod extension 160 is designed to be adjustable. For instance, the interchangeable plunger rod 150 can be designed to fit inside the interchangeable plunger rod extension 160 as shown in FIG. 8(B). The interchangeable plunger rod 150 may comprise one or more sets of tabs for engaging one or more sets of slots in the interchangeable plunger rod extension 160. This makes the combined length of the interchangeable plunger rod 150, interchangeable plunger rod extension 160, and plunger cap 165 adjustable as described in the previous other embodiments. It can also be imagined within the scope of the embodiments that the interchangeable plunger rod 150 could be telescopically designed to fit in or connect to the interchangeable plunger rod extension 160.

| Table - 5 | 1.0mL Capacity Syringe | | | |
|---|---|---|---|---|
| Plunger Cap Length (mm) | 5 | 5 | 5 | 5 |
| Adjustable combined Interchangeable Plunger Rod and Interchangeable Plunger Rod Extension assembly length (mm) | 50 | 58 | 66 | 74 |
| Combined Length (Plunger Cap + Adjustable Interchangeable Plunger Rod length) (mm) | 55 | 63 | 71 | 79 |
| Chamber Capacity Volume (mL) | 1.0 | 1.0 | 1.0 | 1.0 |
| Fill/Solution Volume (mL) | 1.0 | 0.75 | 0.5 | 0.25 |

Table 5 shows the various configurations of the components of the third embodiment of the invention using a 1.0 mL capacity pre-filled syringe 170. In the first embodiment the interchangeable plunger rod 150 length is maintained at a constant 50 mm length, the interchangeable plunger rod extension 160 is changed in length, and the syringe chamber 190 is kept constant at 1.0 mL. This arrangement and orientation allow a manufacturer to quickly and effectively adjust certain components to inject various solution volumes 250 or 250'. As shown in Table 5 it can be observed in a 1.0 mL capacity syringe that various solution volumes 250 of 1.0, 0.75, 0.5, and 0.25 mL can be injected from a 1.0 mL capacity syringe while varying the combined lengths of the interchangeable plunger rod extension 160 and the interchangeable plunger rod 150 at 50, 58, 66, and 74 mm when telescopically sliding the plunger extension 160 along the interchangeable plunger rod 150 and locking at the discrete lengths when the tabs are engaged with the appropriate set of slots. The ability to inject various amounts of solution volume 250 or 250' makes it a very flexible and efficient system without the need for redesigning and redeveloping another version of the AI when a different solution volume 250 or 250' is required.

Table 6 shows similar data and results but using a larger 2.0 mL pre-filled syringe 170 and method. Table 6 shows a 2.0 mL capacity syringe with various solution volumes 250 of 2.0, 1.5, 1.0, and 0.5 mL. The chamber 190 volume is fixed at 2.0 mL, while varying the combined adjustable lengths of the interchangeable plunger rod extension 160 and the interchangeable plunger rod 150 at 50, 60, 70, and 80 mm in the same way as previously described for the 1.0 mL capacity.

| Table -6 | 2.0mL Capacity Syringe | | | |
|---|---|---|---|---|
| Plunger Cap Length (mm) | 5 | 5 | 5 | 5 |
| Adjustable combined Interchangeable Plunger Rod and Interchangeable Plunger Rod Extension Assembly Length (mm) | 50 | 60 | 70 | 80 |
| Combined Length (Plunger Cap and Adjustable Interchangeable Plunger Rod and Interchangeable Plunger Rod Extension Length) (mm) | 55 | 65 | 75 | 85 |
| Chamber Capacity Volume (mL) | 2.0 | 2.0 | 2.0 | 2.0 |
| Fill/Solution Volume (mL) | 2.0 | 1.5 | 1.0 | .5 |

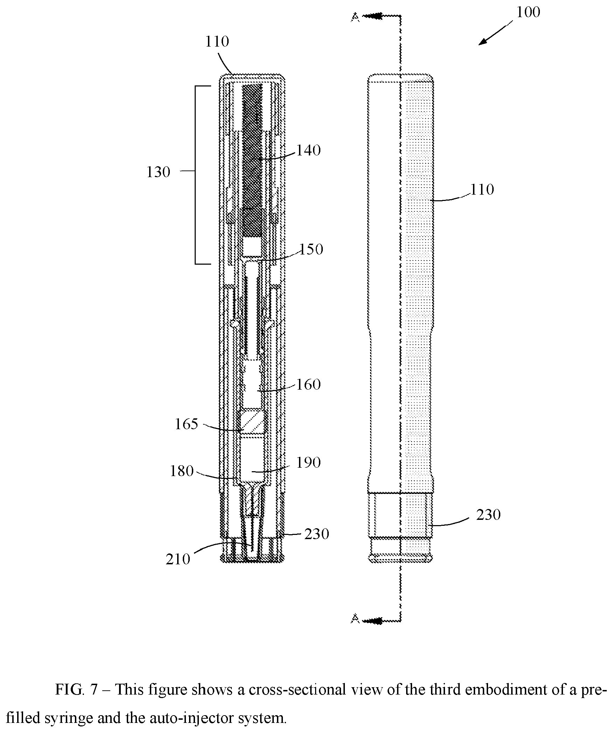

FIG. 7 shows a cross-sectional view of the same third embodiment of the invention.

FIG. 8 shows comparisons of various pre-filled syringes 170 and auto-injector systems 100 with (A) various interchangeable plunger rod extension 160 lengths; and (B) various sliding positions of the interchangeable plunger rod extension 160 along the interchangeable plunger rod 150. As can be seen with each of the embodiments varying the length of the interchangeable plunger rod extension 160 or the interchangeable plunger rod 150 or adjusting the combined length results in a change in the syringe chamber 190 capacity. By varying one or more of these component lengths or adjusting their combined length, the AI can inject any one of a variety of defined volumes from a pre-filled syringe 170 without the need for redesigning and redeveloping another version of the AI when a different solution volume 250 or 250' is required.

FIG 9 shows a method of using the third embodiment to deliver the desired injection volume that may be different from the filled solution volume 250 from a pre-filled syringe. FIG 9 (A) shows how the entire solution volume 250 contained within the syringe chamber 190 can be injected when the combined length of the interchangeable plunger rod 150 and the interchangeable plunger rod extension 160 are at an adjusted length that pushes the plunger cap 165 the entire length of the syringe chamber 190. FIG 9 (B) shows how the solution volume 250 can be reduced from the same pre-filled syringe in FIG 9 (A) when the overall combined adjusted length of the interchangeable plunger rod 150 and interchangeable plunger rod extension 160 is reduced. This reduction in overall length of the adjustable interchangeable plunger rod assembly reduces the interchangeable plunger rod cap's 165 travel distance by the spring 140 because the spring 140 has a set extension distance which results in some solution remaining within the syringe chamber 190 and thus results in less solution volume 250 from being injected. FIG 9 (C) shows how the solution volume 250 to be injected can be further reduced by further shortening the combined length of the interchangeable plunger rod 150 and the interchangeable plunger rod extension 165. Table 7 & 8 compare the combined length of the adjustable interchangeable plunger rod 150 and interchangeable plunger rod extension 160 and plunger cap 165 to the amount of the solution volume 250 that is injected from either a 1.0mL syringe or 2.0mL syringe.

| Table - 7 | 1.0mL Capacity Syringe | | | |
|---|---|---|---|---|
| Plunger Cap Length (mm) | 5 | 5 | 5 | 5 |
| Adjustable combined Interchangeable Plunger Rod and Interchangeable Plunger Rod Extension Assembly Length (mm) | 74 | 66 | 58 | 50 |
| Combined Length (Plunger Cap and Adjustable Interchangeable Plunger Rod and Interchangeable Plunger Rod Extension Length) (mm) | 79 | 71 | 63 | 55 |
| Chamber Capacity Volume (mL) | 1.0 | 1.0 | 1.0 | 1.0 |
| Fill/Solution Volume (mL) | 1.0 | 0.75 | 0.5 | 0.25 |

| Table -8 | 2.0mL Capacity Syringe | | | |
|---|---|---|---|---|
| Plunger Cap Length (mm) | 5 | 5 | 5 | 5 |
| Adjustable combined Interchangeable Plunger Rod and Interchangeable Plunger Rod Extension Length (mm) | 80 | 70 | 60 | 50 |
| Combined Length (Plunger Cap and Adjustable Interchangeable Plunger Rod and Interchangeable Plunger Rod Extension Length) (mm) | 85 | 75 | 65 | 55 |
| Chamber Capacity Volume (mL) | 2.0 | 2.0 | 2.0 | 2.0 |
| Fill/Solution Volume (mL) | 2.0 | 1.5 | 1.0 | .5 |

Having discussed the embodiments of the invention, a description of their assembly is now in order. FIG. 2 shows the component parts and how they are assembled in the first embodiment of the invention.

FIG. 2 shows how the first embodiment of the invention is assembled. The auto-injector housing 110 serves to encase and protect the internal components of the auto-injector system 100. The auto-injector housing 110 has an actuating end and an injection end as show in the figures.

The release mechanism 130 is coaxially aligned and positioned in the auto-injector housing 110 near the actuating end of the housing 110. It slides all the way into the auto-injector housing 110 until it abuts the actuating end of the device. The spring 140 is then co-axially aligned and inserted to abut the release mechanism 130. Depending upon the embodiment either the interchangeable plunger rod 150 or the interchangeable plunger rod extension 160 is co-axially aligned and inserted into the auto-injector housing 110. In the first embodiment the interchangeable plunger rod 150 is co-axially aligned and contacts the spring 140 and interchangeable plunger rod extension 160.

Next, the pre-filled syringe 170 is inserted co-axially into the injection end of the auto-injector housing 110 until the plunger cap 165 abuts the end of the interchangeable plunger rod extension 160 or the interchangeable plunger rod 150. Once all the components have been assembled together and inserted into the auto-injector system 100, the cap 230 can be attached to enclose the auto-injector housing 110 and internal components. This process can be repeated, or parts changed, replaced or adjusted in different injection runs and/or embodiments.

Referring to FIG. 4, the second embodiment of the invention, is assembled similar to the process just described for the first embodiment of the invention. The auto-injector housing 110 serves to enclose and protect the internal components of the auto-injector system 100. The release mechanism 130 is first positioned into the auto-injector housing 110. It slides all the way into the auto-injector housing 110 until it abuts the actuating end of the device. The spring 140 it then inserted to abut the release mechanism 130. Next, the interchangeable plunger rod extension 160 is co-axially aligned and inserted into the auto-injector housing 110. The interchangeable plunger rod 150 is then co-axially aligned and inserted into the auto-injector housing 110. The plunger cap 165 is inserted into the syringe chamber 190 opposite the injection end. The main difference in assembly between this embodiment and the first embodiment is the interchangeable plunger rod extension 160 is inserted first and abuts the spring 140. In other words, the interchangeable plunger rod extension 160 is co-axially aligned and contacts the spring 140 and the interchangeable plunger rod 150.

As previously discussed in FIG. 2, Next, the pre-filled syringe 170 is inserted into the injection end of the auto-injector housing 110 until the plunger cap 165 abuts the end of the interchangeable plunger rod 150. Once all the components have been assembled together and inserted into the auto-injector system 100, the cap 230 can be attached to enclose the auto-injector housing 110 and internal components.

Referring to FIG. 6, the third embodiment of the invention has the interchangeable plunger rod 150 inserted to abut the spring 140 similar to the first embodiment. Further, the interchangeable plunger rod extension 160 is then inserted next, but in this embodiment the interchangeable plunger rod 150 is designed to fit within the interchangeable plunger rod extension 160 such that the interchangeable plunger rod extension 160 is telescopically slidable along a partial length of the interchangeable plunger rod 150. The interchangeable plunger rod 150 is coaxially aligned and fits telescopically within the interchangeable plunger rod extension 160, wherein the interchangeable plunger rod 150 and interchangeable plunger rod extension 160 define an adjustable interchangeable plunger rod assembly which contacts the spring 140 and the plunger cap 165, the adjustable interchangeable plunger rod assembly having a defined length. The remainder of the assembly is similar to each of the embodiments of the invention previously described.

In summary, the embodiments provide an auto-injector system 100 for injecting various solution volumes 250, comprising: an auto-injector housing 110 having an actuating end and an injection end, the housing comprising; a release mechanism 130 co-axially aligned in the housing near the actuating end; a spring 140 co-axially aligned and in contact with the release mechanism 130; a pre-filled syringe 170 co-axially aligned in the housing 110 at the injection end, comprising a set capacity syringe barrel 180 having a syringe chamber 190 for holding a plunger cap 165 with a plunger cap length and a solution volume 250 for injecting, wherein the plunger cap 165 retains the solution volume 250 within the syringe chamber 190; and a syringe needle 210 attached to the syringe barrel 180 for injecting the solution volume 250; an interchangeable plunger rod 150 having a defined interchangeable plunger rod length and being co-axially aligned in the housing 110 between the spring 140 and the plunger cap 165; and an interchangeable plunger rod extension 160 having a defined interchangeable plunger rod extension length and being co-axially aligned and contacting the interchangeable plunger rod 150, wherein the lengths of the spring 140, interchangeable plunger rod 150, interchangeable plunger rod extension 160, and plunger cap 165 define a co-axially aligned and continuous auto-injector system 100 for injecting various solution volumes 250; and an optional auto-inj ector cap 230 for covering the auto-injector housing 110 at the injection end.

Further, the embodiments provide an auto-injector system 100 wherein the interchangeable plunger rod 150 is co-axially aligned and contacts the spring 140 and interchangeable plunger rod extension 160.

Further, the embodiments provide an auto-injector system 100, wherein the pre-filled syringe 170 set capacity of the syringe barrel 180 and syringe chamber 190 is in a range of from 1.0 to 2.0 mL , the interchangeable plunger rod 150 length is in a range of from 5 to 80 mm, the interchangeable plunger rod extension 160 length is in a range of from 5 to 80 mm, the plunger cap 165 length is in a range of from 5 to 6mm, and a solution volume 250 is in the range of from 0.1 to 1.9 mL.

Further, the embodiments provide an auto-injector system 100, wherein the interchangeable plunger rod 150 is replaced with a second interchangeable plunger rod 150', the second interchangeable plunger rod 150' having a length in the range of from 5 to 80 mm, the interchangeable plunger rod extension 160 is replaced with a second interchangeable plunger rod extension 160', the second interchangeable plunger rod extension 160' having a length in a range of from 5 to 80 mm, the plunger cap 165 length is in a range of from 5 to 6mm, and the second solution volume 250' is in the range of 0.1 to 1.9 ml.

Further, the embodiments provide an auto-injector system 100, wherein the interchangeable plunger rod 150 length is 50 mm, the interchangeable plunger rod extension 160 length is 0 mm, the plunger cap 165 length is 5 mm, the solution volume 250 is 1.0 mL, the second interchangeable plunger rod 150' length is 50 mm, the second interchangeable plunger rod extension 160' length is 24 mm, the plunger cap 165 length is 5 mm, and the second solution volume 250' is 0.25 mL.

Further, the embodiments provide an auto-injector system 100, wherein the interchangeable plunger rod extension 160 is co-axially aligned and contacts the spring 140 and the interchangeable plunger rod 150.

Further, the embodiments provide an auto-injector system 100 wherein the pre-filled syringe 170 set capacity of the syringe barrel 180 and syringe chamber 190 is in a range of from 1.0 to 2.0 mL, the interchangeable plunger rod 150 length is in a range of from 5 to 80 mm, the interchangeable plunger rod extension 160 length is in a range of from 5 to 80 mm, the plunger cap 165 length is in a range of from 5 to 6mm, and a solution volume 250 is in the range of from 0.1 to 1.9 mL.

Further, the embodiments provide an auto-injector system 100, wherein the interchangeable plunger rod 150 is replaced with a second interchangeable plunger rod 150', the second interchangeable plunger rod 150' having a length in the range of from 5 to 80 mm, the interchangeable plunger rod extension 160 is replaced with a second interchangeable plunger rod extension 160', the second interchangeable plunger rod extension 160' length is in a range of from 5 to 80 mm, the plunger cap 165 length is in a range of from 5 to 6mm, and the second solution volume 250 is in the range of 0.1 to 1.9 ml.

Further, the embodiments provide an auto-injector system 100, wherein the interchangeable plunger rod 150 length is 40 mm, the interchangeable plunger rod extension 160 length is 10 mm, the plunger cap 165 length is 5 mm, the solution volume 250 of 1.0 mL is injected, the second interchangeable plunger rod 150' length is 64 mm, the second interchangeable plunger rod extension 160' length is 10 mm, the plunger cap 165 length is 5 mm, and the second solution volume 250' is 0.25 mL.

Further, the embodiments provide an auto-injector system 100, wherein the interchangeable plunger rod 150 is coaxially aligned and fits telescopically within the interchangeable plunger rod extension 160, wherein the interchangeable plunger rod 150 and interchangeable plunger rod extension 160 define an adjustable interchangeable plunger rod assembly which contacts the spring 140 and the plunger cap 165, the adjustable interchangeable plunger rod assembly having a length in a range of from 5 to 80 mm, and a solution volume 250 in the range of 0.25 to 1.0 mL.

Further, the embodiments provide an auto-injector system 100, wherein the adjustable interchangeable plunger rod assembly is adjusted to a second adjustable interchangeable plunger rod assembly length and a second solution volume 250' is injected, wherein the second adjustable interchangeable plunger rod length is in a range of from 5 to 80 mm, and the second solution volume injected is in the range of 0.25 to 1.0 mL.

Further, the embodiments provide an auto-injector system 100, wherein the adjustable interchangeable plunger rod assembly length is 50 mm, the plunger cap 165 length is 5 mm, a solution volume 250 of 1.0 mL is injected, the second adjustable interchangeable plunger rod assembly length is 74 mm, the plunger cap length 165 is 5 mm, and the second solution volume 250' injected is 0.25 mL.

Having now described each of the embodiments and their assembly, a description of the method of using the device is now in order.

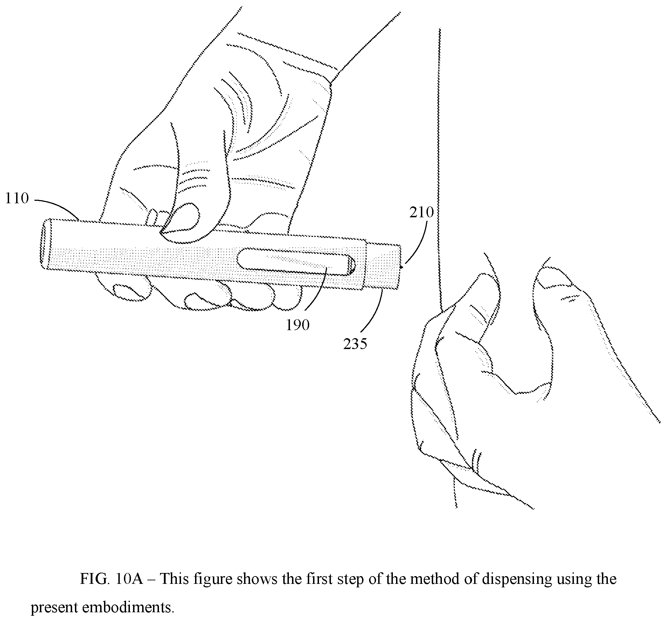

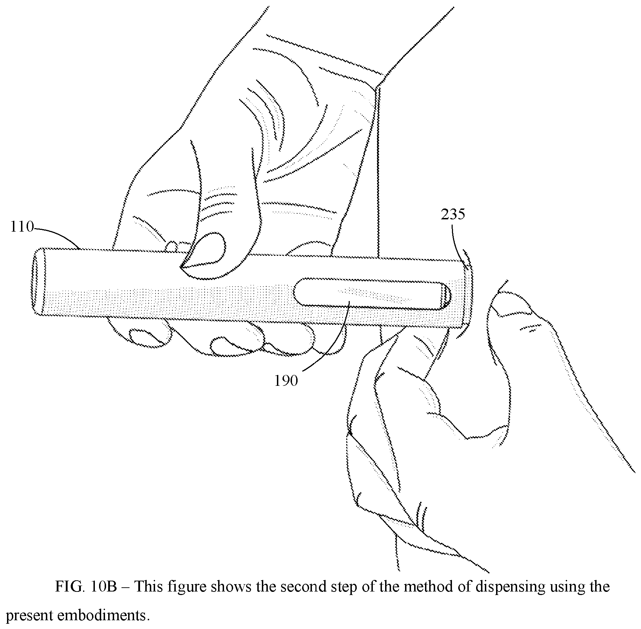

FIG. 10 shows the not-claimed method of using the auto-injector system 100. The optional auto-injector cap 230 and syringe cap 220 are first removed from the auto-injector 100 and pre-filled syringe 170.

The syringe needle 210 is then exposed and ready for use for an injection. The auto-injector 100 is then aligned with the skin surface of the user as shown in FIG. 10A. The auto-injector system's 100 actuator 235 are then pressed against the user's skin. The actuator 235 is continually depressed against the user's skin such that the syringe needle 210 punctures and penetrates the user's skin until the prescribed needle depth is achieved. Next, the release mechanism 130 is triggered by this action to deliver the solution volume 250 or 250' subcutaneously to the user.

Methods for injecting various solution volumes using an auto-injector system 100 according to the invention, comprise: providing an auto-injector system 100 for injecting various solution volumes 250, comprising an auto-injector housing 110 having an actuating end and an injection end, the housing 119 comprising a release mechanism 130 co-axially aligned in the housing near the actuating end; a spring 140 co-axially aligned and in contact with the release mechanism 130; a pre-filled syringe 170 co-axially aligned in the housing 110 at the injection end, comprising a set capacity syringe barrel 180 having a syringe chamber 190 for holding a plunger cap 165 with a plunger cap length and a solution volume 250, wherein the plunger cap 165 retains the solution volume 250 within the syringe chamber 190; and a syringe needle 210 attached to the syringe barrel 190 for injecting the solution volume 250; an interchangeable plunger rod 150 that's interchangeable having a defined interchangeable plunger rod 150 length and being co-axially aligned in the housing 110 between the spring 140 and the plunger cap 165; and an interchangeable plunger rod extension 160 that's interchangeable having a defined interchangeable plunger rod extension 160 length and being co-axially aligned and contacting the interchangeable plunger rod 150, wherein the lengths of the spring 140, interchangeable plunger rod 150, interchangeable plunger rod extension 160, and plunger cap 165 define a co-axially aligned and continuous auto-injector system 100 for injecting various solution volumes 250 or 250'; and an optional auto-injector cap 230 for covering the auto-injector housing 110 at the injection end; and injecting the various solution volumes 250 or 250'.

Said methods further comprise replacing the interchangeable plunger rod extension 160 with a second interchangeable plunger rod extension 160' having a second interchangeable plunger rod extension 160' length and injecting a second solution volume 250'.

Said methods further comprise replacing the interchangeable plunger rod 150 with a second interchangeable plunger rod 150' having a second interchangeable plunger rod 150' length and injecting a second solution volume 250'.

Said methods wherein only a portion of the solution volume 250 or 250' in the syringe chamber 190 is injected.

Said methods where only a portion of the solution volume 250 or 250' from the syringe chamber 190 is injected when the plunger cap 165 is initially positioned at the end of the syringe chamber 190 closer to the actuating end of the auto-injector housing 110 and then only partially compressed in the syringe chamber 190 toward the injection end.

## Claims

1. An auto-injector system (100) for injecting a predetermined solution volume in one shot from a pre-filled syringe (170), comprising:
(a) an auto-injector housing (110) having an actuating end and an injection end, the housing (110) comprising;
(i) a release mechanism (130) co-axially aligned in the housing (110) near the actuating end;
(ii) a spring (140) co-axially aligned and in contact with the release mechanism (130);
(iii) a pre-filled syringe (170) co-axially aligned in the housing (110) at the injection end, comprising a set capacity syringe barrel (180) having a syringe chamber (190) for holding a plunger cap (165) with a plunger cap length and a solution volume (250) for injecting, wherein the plunger cap (165) retains the solution volume (250) within the syringe chamber (190); and a syringe needle (210) attached to the syringe barrel (180) for injecting the predetermined solution volume;
(iv) an interchangeable plunger rod (150) having a defined interchangeable plunger rod length and being co-axially aligned in the housing (110) between the spring (140) and the plunger cap (165); and
(v) an interchangeable plunger rod extension (160) having a defined interchangeable plunger rod extension length and being co-axially aligned in the housing (110) between the spring (140) and the plunger cap (165) and either
a. contacting the interchangeable plunger rod (150) and the spring (140); or
b. contacting the plunger cap (165) and the interchangeable plunger rod (150),
wherein the lengths of the spring (140), interchangeable plunger rod (150), interchangeable plunger rod extension (160), and plunger cap (165) define the predetermined solution volume which is injected from the pre-filled syringe (170) in the one shot, and
wherein said predetermined solution volume can be varied by varying the interchangeable plunger rod length, the interchangeable plunger rod extension length, or both; and
(b) an optional auto-injector cap (230) for covering the auto-injector housing (110) at the injection end.

2. An auto-injector system (100) as recited in claim 1, wherein the interchangeable plunger rod (150) contacts the spring (140) and interchangeable plunger rod extension (160).

3. An auto-injector system (100) as recited in claim 2, wherein in a first injection run the pre-filled syringe set capacity of the syringe barrel (180) and syringe chamber (190) is in a range of from 1.0 to 2.0 mL, the interchangeable plunger rod length is in a range of from 5 to 80 mm, the interchangeable plunger rod extension length is in a range of from 5 to 80 mm, the plunger cap length is in a range of from 5 to 6 mm, and predetermined the solution volume to be injected is in the range of from 0.1 to 1.9 mL.

4. An auto-injector system (100) as recited in claim 3, wherein in a second injection run, the interchangeable plunger rod (150) is not replaced, the interchangeable plunger rod (150) having a length in the range of from 5 to 80 mm, the interchangeable plunger rod extension (160) is replaced with a second interchangeable plunger rod extension (160), the second interchangeable plunger rod extension (160) having a length in a range of from 5 to 80 mm, the plunger cap (165) is not replaced, the plunger cap length is in a range of from 5 to 6 mm, and the second predetermined solution volume to be injected is in the range of 0.1 to 1.9 ml.

5. An auto-injector system (100) as recited in claim 4, wherein the interchangeable plunger rod length is 50 mm, the interchangeable plunger rod extension length is 0 mm, the plunger cap length is 5 mm, the solution volume is 1.0 mL, the second interchangeable plunger rod extension length is 24 mm, and the second predetermined solution volume to be injected is 0.25 mL.

6. An auto-injector system (100) as recited in claim 1, wherein the interchangeable plunger rod extension (160) contacts the spring (140) and the interchangeable plunger rod (150).

7. An auto-injector system (100) as recited in claim 6, wherein in the first injection run the pre-filled syringe set capacity of the syringe barrel (180) and syringe chamber (190) is in a range of from 1.0 to 2.0 mL, the interchangeable plunger rod length is in a range of from 5 to 80 mm, the interchangeable plunger rod extension length is in a range of from 5 to 80 mm, the plunger cap length is in a range of from 5 to 6 mm, and a predetermined solution volume to be injected is in the range of from 0.1 to 1.9 mL.

8. An auto-injector system (100) as recited in claim 7, wherein in a second injection run the interchangeable plunger rod (150) is replaced with a second interchangeable plunger rod (150), the second interchangeable plunger rod (150) having a length in the range of from 5 to 80 mm, the interchangeable plunger rod extension (160) remains the same and is in a range of from 5 to 80 mm, the plunger cap (165) remains the same length and is in a range of from 5 to 6 mm, and the second predetermined solution volume to be injected is in the range of 0.1 to 1.9 ml.

9. An auto-injector system (100) as recited in claim 8, wherein the interchangeable plunger rod length is 40 mm, the interchangeable plunger rod extension length is 10 mm, the plunger cap length is 5 mm, the predetermined solution volume to be injected is 1.0 mL, the second interchangeable plunger rod length is 64 mm, the interchangeable plunger rod extension length is 10 mm, the plunger cap length is 5 mm, and the second solution volume to be injected is 0.25 mL.

10. An auto-injector system (100) as recited in claim 1, wherein the interchangeable plunger rod (250)fits telescopically within the interchangeable plunger rod extension (160), wherein the interchangeable plunger rod (150) and interchangeable plunger rod extension (160) define an adjustable interchangeable plunger rod assembly which contacts the spring and the plunger cap, the adjustable interchangeable plunger rod assembly having a length in a range of from 5 to 80 mm, and a predetermined solution volume to be injected in the range of 1.0 to 0.25 mL.

11. An auto-injector system (100) as recited in claim 10, wherein the adjustable interchangeable plunger rod assembly length is adjusted to a second adjustable interchangeable plunger rod assembly length and a second predetermined solution volume is injected, wherein the second adjustable interchangeable plunger rod assembly length is in a range of from 5 to 80 mm, and the second solution volume is in the range of 1.0 to 0.25 mL.

12. An auto-injector system (100) as recited in claim 11, wherein the adjustable interchangeable plunger rod assembly length is 50 mm, the plunger cap length is 5 mm, the solution volume is 1.0 mL, the second adjustable interchangeable plunger rod assembly length is 74 mm, the plunger cap length is 5 mm, and the second predetermined solution volume is 0.25 mL.

## Patentansprüche

1. Ein Autoinjektionssystem (100) zum Injizieren eines vorbestimmten Lösungsvolumens in einem Schuss aus einer vorgefüllten Spritze (170), umfassend:
(a) ein Autoinjektorgehäuse (110) mit einem Betätigungsende und einem Injektionsende, wobei das Gehäuse (110) umfasst;
(i) einen Freisetzungsmechanismus (130), der koaxial im Gehäuse (110) in der Nähe des Betätigungsendes ausgerichtet ist;
(ii) eine Feder (140), die koaxial ausgerichtet ist und in Kontakt mit dem Freisetzungsmechanismus (130) steht;
(iii) eine vorgefüllte Spritze (170), die koaxial in dem Gehäuse (110) an dem Injektionsende ausgerichtet ist und einen Spritzenzylinder (180) mit festgelegter Kapazität umfasst, der eine Spritzenkammer (190) zum Halten einer Kolbenkappe (165) mit einer Kolbenkappenlänge und einem Lösungsvolumen (250) zum Injizieren aufweist, wobei die Kolbenkappe (165) das Lösungsvolumen (250) innerhalb der Spritzenkammer (190) zurückhält; und eine Spritzennadel (210), die an dem Spritzenzylinder (180) zum Injizieren des vorbestimmten Lösungsvolumens angebracht ist;
(iv) eine austauschbare Kolbenstange (150), die eine definierte austauschbare Kolbenstangenlänge aufweist und koaxial im Gehäuse (110) zwischen der Feder (140) und der Kolbenkappe (165) ausgerichtet ist; und
(v) eine austauschbare Kolbenstangenverlängerung (160), die eine definierte Länge der austauschbaren Kolbenstangenverlängerung aufweist und koaxial im Gehäuse (110) zwischen der Feder (140) und der Kolbenkappe (165) ausgerichtet ist und entweder
a. die austauschbare Kolbenstange (150) und die Feder (140) berührt; oder
b. die Kolbenkappe (165) und die austauschbare Kolbenstange (150) berührt,
wobei die Längen der Feder (140), der austauschbaren Kolbenstange (150), der austauschbaren Kolbenstangenverlängerung (160) und der Kolbenkappe (165) das vorbestimmte Lösungsvolumen definieren, das aus der vorgefüllten Spritze (170) mit einem Schuss injiziert wird, und
wobei das vorbestimmte Lösungsvolumen durch Variieren der Länge der austauschbaren Kolbenstange, der Länge der austauschbaren Kolbenstangenverlängerung oder beider variiert werden kann; und
(b) eine optionale Autoinjektor-Kappe (230) zum Abdecken des Autoinjektorgehäuses (110) am Injektionsende.

2. Autoinjektionssystem (100) nach Anspruch 1, wobei die austauschbare Kolbenstange (150) die Feder (140) und die austauschbare Kolbenstangenverlängerung (160) berührt.

3. Autoinjektionssystem (100) nach Anspruch 2, wobei in einem ersten Injektionslauf die Kapazität der vorgefüllten Spritze, des Spritzenzylinders (180) und der Spritzenkammer (190) in einem Bereich von 1,0 bis 2,0 ml liegt, die Länge der austauschbaren Kolbenstange in einem Bereich von 5 bis 80 mm liegt, die Länge der Kolbenkappe in einem Bereich von 5 bis 6 mm liegt und das vorbestimmte zu injizierende Lösungsvolumen in einem Bereich von 0,1 bis 1,9 ml liegt.

4. Autoinjektionssystem (100) nach Anspruch 3, wobei in einem zweiten Injektionslauf die austauschbare Kolbenstange (150) nicht ausgetauscht wird, die austauschbare Kolbenstange (150) eine Länge im Bereich von 5 bis 80 mm aufweist, die austauschbare Kolbenstangenverlängerung (160) durch eine zweite austauschbare Kolbenstangenverlängerung (160) ersetzt wird, die zweite austauschbare Kolbenstangenverlängerung (160) eine Länge in einem Bereich von 5 bis 80 mm aufweist, die Kolbenkappe (165) nicht ausgetauscht wird, die Länge der Kolbenkappe in einem Bereich von 5 bis 6 mm liegt und das zweite vorbestimmte, zu injizierende Lösungsvolumen im Bereich von 0,1 bis 1,9 ml liegt.

5. Autoinjektionssystem (100) nach Anspruch 4, wobei die Länge der austauschbaren Kolbenstange 50 mm, die Länge der austauschbaren Kolbenstangenverlängerung 0 mm, die Länge der Kolbenkappe 5 mm, das Lösungsvolumen 1,0 mL, die Länge der zweiten austauschbaren Kolbenstangenverlängerung 24 mm und das zweite vorbestimmte zu injizierende Lösungsvolumen 0,25 mL betragen.

6. Autoinjektionssystem (100) nach Anspruch 1, wobei die austauschbare Kolbenstangenverlängerung (160) die Feder (140) und die austauschbare Kolbenstange (150) berührt.

7. Autoinjektionssystem (100) nach Anspruch 6, wobei beim ersten Injektionslauf die Kapazität der vorgefüllten Spritze, des Spritzenzylinders (180) und der Spritzenkammer (190) in einem Bereich von 1,0 bis 2,0 ml liegt, die Länge der austauschbaren Kolbenstange in einem Bereich von 5 bis 80 mm liegt, die Länge der austauschbaren Kolbenstangenverlängerung in einem Bereich von 5 bis 80 mm liegt, die Länge der Kolbenkappe in einem Bereich von 5 bis 6 mm liegt und ein vorbestimmtes, zu injizierendes Lösungsvolumen in einem Bereich von 0,1 bis 1,9 ml liegt.

8. Autoinjektionssystem (100) nach Anspruch 7, wobei in einem zweiten Injektionslauf die austauschbare Kolbenstange (150) durch eine zweite austauschbare Kolbenstange (150) ersetzt wird, wobei die zweite austauschbare Kolbenstange (150) eine Länge im Bereich von 5 bis 80 mm aufweist, die austauschbare Kolbenstangenverlängerung (160) gleich bleibt und in einem Bereich von 5 bis 80 mm liegt, die Kolbenkappe (165) gleich bleibt und in einem Bereich von 5 bis 6 mm liegt, und das zweite vorbestimmte, zu injizierende Lösungsvolumen im Bereich von 0,1 bis 1,9 ml liegt.

9. Autoinjektionssystem (100) nach Anspruch 8, wobei die Länge der austauschbaren Kolbenstange 40 mm, die Länge der austauschbaren Kolbenstangenverlängerung 10 mm, die Länge der Kolbenkappe 5 mm, das vorbestimmte zu injizierende Lösungsvolumen 1,0 ml, die Länge der zweiten austauschbaren Kolbenstange 64 mm, die Länge der austauschbaren Kolbenstangenverlängerung 10 mm, die Länge der Kolbenkappe 5 mm und das zweite zu injizierende Lösungsvolumen 0,25 ml betragen.

10. Autoinjektionssystem (100) nach Anspruch 1, wobei die austauschbare Kolbenstange (150) teleskopisch in die austauschbare Kolbenstangenverlängerung (160) passt, wobei die austauschbare Kolbenstange (150) und die austauschbare Kolbenstangenverlängerung (160) eine einstellbare austauschbare Kolbenstangenbaugruppe definieren, die die Feder und die Kolbenkappe berührt, wobei die einstellbare austauschbare Kolbenstangenbaugruppe eine Länge in einem Bereich von 5 bis 80 mm und ein vorbestimmtes, zu injizierendes Lösungsvolumen in dem Bereich von 1,0 bis 0,25 mL aufweist.

11. Autoinjektionssystem (100) nach Anspruch 10, wobei die einstellbare, austauschbare Kolbenstangenlänge auf eine zweite einstellbare, austauschbare Kolbenstangenlänge eingestellt wird und ein zweites vorbestimmtes Lösungsvolumen injiziert wird, wobei die zweite einstellbare, austauschbare Kolbenstangenlänge in einem Bereich von 5 bis 80 mm liegt und das zweite Lösungsvolumen im Bereich von 1,0 bis 0,25 ml liegt.

12. Autoinjektionssystem (100) nach Anspruch 11, wobei die Länge der einstellbaren austauschbaren Kolbenstange 50 mm, die Länge der Kolbenkappe 5 mm, das Lösungsvolumen 1,0 mL, die Länge der zweiten einstellbaren austauschbaren Kolbenstange 74 mm, die Länge der Kolbenkappe 5 mm und das zweite vorbestimmte Lösungsvolumen 0,25 mL beträgt.

## Revendications

1. Un système d'auto-injecteur (100) permettant d'injecter un volume de solution prédéterminé en une seule fois à partir d'une seringue pré-remplie (170), comprenant :
(a) un boîtier d'auto-injecteur (110) comportant une extrémité d'actionnement et une extrémité d'injection, le boîtier (110) comprenant :
(i) un mécanisme de déclenchement (130) aligné de manière coaxiale dans le boîtier (110) près de l'extrémité d'actionnement ;
(ii) un ressort (140) aligné de manière coaxiale et en contact avec le mécanisme de déclenchement (130) ;
(iii) une seringue pré-remplie (170) coaxialement alignée dans le boîtier (110) à l'extrémité d'injection, comprenant un corps de seringue de capacité déterminée (180) ayant une chambre de seringue (190) pour contenir un capuchon de piston (165) avec une longueur de capuchon de piston et un volume de solution (250) pour l'injection, dans lequel le capuchon de piston (165) retient le volume de solution (250) à l'intérieur de la chambre de seringue (190) ; et une aiguille de seringue (210) attachée au corps de seringue (180) pour injecter le volume de solution prédéterminé ;
(iv) une tige de piston interchangeable (150) ayant une longueur de tige de piston interchangeable définie et étant alignée coaxialement dans le boîtier (110) entre le ressort (140) et le capuchon de piston (165) ; et
(v) une extension de tige de piston interchangeable (160) ayant une longueur définie d'extension de tige de piston interchangeable et étant alignée coaxialement dans le boîtier (110) entre le ressort (140) et le capuchon de piston (165) et soit
a. en contact avec la tige de piston interchangeable (150) et le ressort (140) ; ou
b. en contact avec le capuchon de piston (165) et la tige de piston interchangeable (150),
en ce que les longueurs du ressort (140), de la tige de piston interchangeable (150), de l'extension de la tige de piston interchangeable (160) et du capuchon de piston (165) définissent le volume de solution prédéterminé qui est injecté à partir de la seringue pré-remplie (170) en une seule fois, et
**caractérisé en ce que** ledit volume de solution prédéterminé peut être modifié en changeant la longueur de la tige de piston interchangeable, la longueur de l'extension de la tige de piston interchangeable, ou les deux ; et
(b) un capuchon d'auto-injecteur optionnel (230) pour couvrir le boîtier de l'auto-injecteur (110) à l'extrémité d'injection.

2. Système d'auto-injecteur (100) selon la revendication 1, dans lequel la tige de piston interchangeable (150) entre en contact avec le ressort (140) et l'extension de tige de piston interchangeable (160).

3. Système d'auto-injecteur (100) selon la revendication 2, dans lequel, lors d'une première injection, la capacité de la seringue pré-remplie du corps de la seringue (180) et de la chambre de la seringue (190) est comprise entre 1,0 et 2,0 mL, la longueur de la tige de piston interchangeable est comprise entre 5 et 80 mm, la longueur du capuchon de piston est comprise entre 5 et 6 mm, et le volume prédéterminé de la solution à injecter est compris entre 0,1 et 1,9 mL.

4. Système d'auto-injecteur (100) selon la revendication 3, dans lequel, lors d'une deuxième injection, la tige de piston interchangeable (150) n'est pas remplacée, la tige de piston interchangeable (150) ayant une longueur comprise entre 5 et 80 mm, l'extension de tige de piston interchangeable (160) est remplacée par une seconde extension de tige de piston interchangeable (160), la seconde extension de tige de piston interchangeable (160) ayant une longueur comprise entre 5 et 80 mm, le capuchon de piston (165) n'est pas remplacé, la longueur du capuchon de piston est comprise entre 5 et 6 mm, et le second volume de solution prédéterminé à injecter est compris entre 0,1 et 1,9 ml.

5. Système d'auto-injecteur (100) selon la revendication 4, dans lequel la longueur de la tige de piston interchangeable est de 50 mm, la longueur de l'extension de la tige de piston interchangeable est de 0 mm, la longueur du capuchon de piston est de 5 mm, le volume de la solution est de 1,0 mL, la deuxième longueur de l'extension de la tige de piston interchangeable est de 24 mm, et le deuxième volume prédéterminé de la solution à injecter est de 0,25 mL.

6. Système d'auto-injecteur (100) selon la revendication 1, dans lequel l'extension de tige de piston interchangeable (160) entre en contact avec le ressort (140) et la tige de piston interchangeable (150).

7. Système d'auto-injecteur (100) selon la revendication 6, dans lequel, lors de la première injection, la capacité de la seringue pré-remplie du corps de la seringue (180) et de la chambre de la seringue (190) est comprise entre 1,0 et 2,0 mL, la longueur de la tige de piston interchangeable est comprise entre 5 et 80 mm, la longueur de l'extension de la tige de piston interchangeable est comprise entre 5 et 80 mm, la longueur du capuchon de piston est comprise entre 5 et 6 mm, et un volume de solution prédéterminé à injecter est compris entre 0,1 et 1,9 mL.

8. Système d'auto-injecteur (100) selon la revendication 7, dans lequel, lors d'une deuxième injection, la tige de piston interchangeable (150) est remplacée par une deuxième tige de piston interchangeable (150), la deuxième tige de piston interchangeable (150) ayant une longueur comprise entre 5 et 80 mm, l'extension de la tige de piston interchangeable (160) reste la même et est comprise entre 5 et 80 mm, le capuchon de piston (165) reste de la même longueur et est compris entre 5 et 6 mm, et le second volume de solution prédéterminé à injecter est compris entre 0,1 et 1,9 ml.

9. Système d'auto-injecteur (100) selon la revendication 8, dans lequel la longueur de la tige de piston interchangeable est de 40 mm, la longueur de l'extension de la tige de piston interchangeable est de 10 mm, la longueur du capuchon de piston est de 5 mm, le volume de solution prédéterminé à injecter est de 1,0 mL, la deuxième longueur de la tige de piston interchangeable est de 64 mm, la longueur de l'extension de la tige de piston interchangeable est de 10 mm, la longueur du capuchon de piston est de 5 mm, et le deuxième volume de solution à injecter est de 0,25 mL.

10. Système d'auto-injecteur (100) selon la revendication 1, dans lequel la tige de piston interchangeable (250) s'adapte de manière télescopique à l'intérieur de l'extension de tige de piston interchangeable (160), dans lequel la tige de piston interchangeable (150) et l'extension de tige de piston interchangeable (160) définissent un ensemble de tige de piston interchangeable réglable qui entre en contact avec le ressort et le capuchon de piston, l'ensemble de tige de piston interchangeable réglable ayant une longueur dans une plage de 5 à 80 mm, et un volume de solution prédéterminé à injecter dans une plage de 1,0 à 0,25 mL.

11. Système d'auto-injecteur (100) selon la revendication 10, dans lequel la longueur de l'ensemble de tige de piston interchangeable réglable est ajustée à une deuxième longueur d'ensemble de tige de piston interchangeable réglable et un deuxième volume de solution prédéterminé est injecté, dans lequel la deuxième longueur d'ensemble de tige de piston interchangeable réglable est comprise entre 5 et 80 mm, et le deuxième volume de solution est compris entre 1,0 et 0,25 mL.

12. Système d'auto-injecteur (100) selon la revendication 11, dans lequel la longueur de l'ensemble de tige de piston interchangeable réglable est de 50 mm, la longueur du capuchon de piston est de 5 mm, le volume de la solution est de 1,0 mL, la deuxième longueur de l'ensemble de tige de piston interchangeable réglable est de 74 mm, la longueur du capuchon de piston est de 5 mm et le deuxième volume prédéterminé de la solution est de 0,25 mL.
